# EUROPEAN PATENT SPECIFICATION

(11) **EP 4 051 108 B1**
(45) Date of publication and mention of the grant of the patent: **15.04.2026**
(21) Application number: 20804128.5
(22) Date of filing: 22.10.2020
(51) Int. Cl.: A61M 25/06, A61M 39/06, A61M 39/10, A61M 39/02, A61B 5/15, A61B 5/154

(54) **BLOOD COLLECTION SYSTEM WITH USER-ADJUSTED PRESSURE MANAGEMENT**
BLUTENTNAHMESYSTEM MIT BENUTZERGESTEUERTEM DRUCKMANAGEMENT
SYSTÈME DE COLLECTE DE SANG AVEC GESTION DE LA PRESSION AJUSTÉE À L'UTILISATEUR

(30) Priority: 30.10.2019 US 201962928102 P; 20.10.2020 US 202017075420
(43) Date of publication of application: 07.09.2022
(73) Proprietor: Becton, Dickinson and Company, Franklin Lakes, NJ 07417 (US)
(72) Inventor: BURKHOLZ, Jonathan Karl, Salt Lake City, Utah 84108 (US); SCHERICH, Megan, Salt Lake City, Utah 84102 (US); BLANCHARD, Curtis H., Riverton, Utah 84096 (US); ISAACSON, S. Ray, Layton, Utah 84041 (US); TRAN, Huy, Riverton, Utah 84065 (US)
(74) Representative: dompatent
(86) International application number: PCT/US2020/056923
(87) International publication number: WO 2021/086722

(56) References cited:
- WO-A1-2013/028759
- WO-A1-88/05286
- WO-A1-99/48425
- CN-U- 203 634 540
- US-A- 4 333 479
- US-A- 4 409 991
- US-A1- 2014 042 094
- US-A1- 2019 282 150
- US-A1- 2019 321 595

## Description

### BACKGROUND

Intravenous catheters are commonly used for a variety of infusion therapies. For example, intravenous catheters may be used for infusing fluids, such as normal saline solution, various medicaments, and total parenteral nutrition, into a patient. Intravenous catheters may also be used for withdrawing blood from the patient.

Common types of intravenous catheter are peripheral IV catheters ("PIVCs"), peripherally inserted central catheters ("PICCs"), and midline catheters. Intravenous catheters may include "over-the needle" catheters, which may be mounted over a needle having a sharp distal tip. The sharp distal tip may be used to pierce skin and the vasculature of the patient. Insertion of the intravenous catheter into the vasculature may follow the piercing of the vasculature by the needle. The needle and the intravenous catheter are generally inserted at a shallow angle through the skin into the vasculature of the patient with a bevel of the needle facing up and away from the skin of the patient.

In order to verify proper placement of the introducer needle and/or the intravenous catheter in the vasculature, a user generally confirms that there is flashback of blood, which may be visible to the user. In some instances, the introducer needle may include a notch disposed towards a distal end of the introducer needle, and in response to the distal tip of the introducer needle being positioned within the vasculature, blood may flow proximally through a needle lumen, exit the needle lumen through the notch, and then travel proximally between an outer surface of the introducer needle and an inner surface of the intravenous catheter.

Accordingly, where the intravenous catheter is at least partially transparent, the user may visualize a small amount of blood "flashback" and thereby confirm placement of the intravenous catheter within the vasculature. Presence of a vasculature entrance indicator, such as flashback, may facilitate successful placement of intravenous catheters. Once placement of the introducer needle within the vasculature has been confirmed, the user may temporarily occlude flow in the vasculature and withdraw the introducer needle, leaving the intravenous catheter in place for future blood withdrawal and/or fluid infusion.

For blood withdrawal, an evacuated blood collection tube may be used. An evacuated blood collection tube includes a test tube with a rubber stopper at one end. The evacuated blood collection tube has had all or a portion of air removed from the test tube so pressure within the evacuated blood collection tube is lower than ambient pressure. Such an evacuated blood collection tube is often referred to as an internal vacuum or a vacuum tube. A commonly used evacuated blood collection tube is a VACUTAINER blood collection tube, available from Becton Dickinson & Company.

To collect a blood sample from a patient, a user first gains access to the patient's vein with either a needle or the intravenous catheter. An adapter is coupled to the needle or the intravenous catheter. The adapter includes an additional needle that penetrates the rubber stopper of the evacuated blood collection tube. When the rubber stopper is penetrated, a pressure in the vein is higher than a pressure in the evacuated blood collection tube, which pushes blood into the evacuated blood collection tube, thus filling the evacuated blood collection tube with blood. A vacuum within the evacuated blood collection tube decreases as the evacuated blood collection tube fills until the pressure in the evacuated blood collection tube equalizes with the pressure in the vein, and the flow of blood stops.

Unfortunately, as blood is drawn into the evacuated blood collection tube, red blood cells are in a high shear stress state and susceptible to hemolysis due to a high initial pressure differential between the vein and the evacuated blood collection tube. Hemolysis may result in rejection and discard of a blood sample. The high initial pressure differential can also result in catheter tip collapse, vein collapse, or other complications that prevent or restrict blood from filling the evacuated blood collection tube.

The subject matter claimed herein is not limited to embodiments that solve any disadvantages or that operate only in environments such as those described above. Rather, this background is only provided to illustrate one example technology area where some implementations described herein may be practiced.

A blood collection system having the features defined within the preamble of claim 1 is described in US2014/042094.

### SUMMARY

The present disclosure relates generally to a blood collection system with useradjusted pressure management, as well as related device. The blood collection according to the present invention is defined by the features of claim 1. Preferred embodiments of the present invention are defined within the dependent claims. A blood collection system according to the invention includes a needle configured to receive an evacuated blood collection tube. In some embodiments, the blood collection system may include a blood collection tube receptacle, which may surround the needle. In some embodiments, the blood collection system may include an adapter configured to couple to a catheter assembly. The blood collection system according to the invention includes a flow regulator disposed between the needle and the adapter. The flow regulator is configured to regulate flow through a fluid pathway of the blood collection system, which extends between the needle and the adapter.

In some embodiments, the adapter may be coupled to the catheter assembly. In some embodiments, the catheter assembly may include a catheter adapter, which may include a distal end, a proximal end, and a lumen extending through the distal end of the catheter adapter and the proximal end of the catheter adapter. In some embodiments, the catheter assembly may include a catheter extending distally from the distal end of the catheter adapter. In some embodiments, the catheter may be inserted into a vein of a patient.

In some embodiments, the flow regulator may be adjusted to reduce an inner diameter of a portion of the fluid pathway extending through the flow regulator to be less than an inner diameter of the catheter. In some embodiments, after adjusting the flow regulator to reduce the inner diameter of the portion of the fluid pathway, the needle may be inserted into the evacuated blood collection tube. In some embodiments, when the evacuated blood collection tube is coupled to the blood collection system, blood may flow more slowly than it otherwise would due to the reduced inner diameter and restriction of the fluid pathway. When the evacuated blood collection tube is coupled to the blood collection system, a high pressure differential between the evacuated blood collection tube and the vein generally creates a risk of hemolysis. However, in some embodiments, the decrease of blood flow due to the reduced inner diameter and restriction of the fluid pathway may decrease the risk of hemolysis. In some embodiments, the decrease of blood flow may also reduce a risk of collapse of the vein and/or the catheter.

In some embodiments, as the evacuated blood collection tube fills with blood, a vacuum within the evacuated blood collection tube decreases and the pressure differential between the evacuated blood collection tube and the vein decreases. In some embodiments, the decreased pressure differential may result in the evacuated blood collection tube filling more slowly over time. In some embodiments, in response to the evacuated blood collection tube partially filling with blood, the flow regulator may be adjusted to increase the inner diameter of the fluid pathway extending through the flow regulator, which may increase blood flow and speed blood collection. In some embodiments, the inner diameter may be increased to be larger than the inner diameter when the needle is inserted into the evacuated blood collection tube. In some embodiments, the inner diameter may be increased to be equal to (or greater) than the inner diameter of the catheter.

The flow regulator according to the invention includes a rotary element. In response to the rotary element being in a first position, a portion of the fluid pathway of the blood collection system may extend through the rotary element. In response to rotation of the rotary element from the first position to a second position, a diameter of the portion of the fluid pathway extending through the rotary element changes. In some embodiments, the rotary element may include one or more holes configured to align with the fluid pathway of the blood collection system. In some embodiments, each of the holes may have a different diameter.

In some embodiments, the rotary element may include a curved slot extending through the rotary element. In some embodiments, an entirety of the curved slot may be configured to align with the fluid pathway of the blood collection system. In some embodiments, a width of the curved slot may continuously increase such that fluid flow through the rotary element continuously changes as the rotary element is rotated.

In some embodiments, the flow regulator may include a stopcock, a squeeze valve, or a slide valve. In some embodiments, the slide valve may include a housing and a body slidable with respect to the housing between a first position and a second position. In some embodiments, the housing may include a first end, a second end, and a lumen extending through the first end and the second end. In some embodiments, the first end of the housing may include an opening. In some embodiments, an outer surface of the body may include one or more channels. In some embodiments, in response to the body being in the first position, blood is configured to flow through a gap between an outer surface of the body and the housing and through the opening. In some embodiments, in response to the housing being in the second position, blood is configured to flow through at least one of the channels and through the opening but may not flow through the gap.

In some embodiments, the blood collection system may include tubing, which may extend between the adapter and the needle. In some embodiments, the flow regulator may include a clamp disposed on the tubing. In some embodiments, the clamp on the tubing may include a roller clamp or a slide clamp. In some embodiments, an inner surface of the tubing may include one or more ribs, which may extend generally parallel to a longitudinal axis of the tubing.

In some embodiments, the slide clamp may include a housing, which may include a slot. In some embodiments, the slide clamp may include a body, which may extend through the slot. In some embodiments, the body may be slidable with respect to the housing between a first position and a second position. In some embodiments, a bottom of the body may include a first surface, a second surface, and a third surface disposed between the first surface and the second surface. In some embodiments, the first surface and the second surface may be generally parallel to the longitudinal axis of the tubing. In some embodiments, the third surface may be angled with respect to the longitudinal axis of the tubing. In some embodiments, the second surface may be closer to the tubing than the first surface.

In some embodiments, the slide clamp may include a roller pin disposed between the body and the tubing. In some embodiments, in response to the body being in the first position, the roller pin may contact the first surface and the tubing. In some embodiments, in response to the body sliding from the first position to the second position, the roller pin moves from the first surface along the third surface to the second surface and decreases fluid flow through the tubing.

In some embodiments, a particular blood collection tube holder may include one or more of the following: a needle configured to receive an evacuated collection tube; a cavity; a tubing disposed within the cavity and in fluid communication with the needle; a button disposed within the cavity; and a spring. In some embodiments, the button may include an opening extending through the button. In some embodiments, the button may include a rib proximate the opening. In some embodiments, the tubing may extend through the opening.

In some embodiments, the spring may be disposed between the button and a wall of the cavity. In some embodiments, the spring may bias the rib against the tubing to compress the tubing. In some embodiments, in response to the button being depressed and the spring being compressed, the tubing may be uncompressed.

It is to be understood that both the foregoing general description and the following detailed description are examples and explanatory and are not restrictive of the invention, as claimed. It should be understood that the various embodiments are not limited to the arrangements and instrumentality shown in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

### BRIEF DESCRIPTION OF THE SEVERAL VIEWS OF THE DRAWINGS

Example embodiments will be described and explained with additional specificity and detail through the use of the accompanying drawings in which:
Figure 1A is an upper perspective view of an example blood collection system, according to some embodiments;
Figure 1B is a cross-sectional view of the blood collection system, illustrating an example rotary element in a first position, according to some embodiments;
Figure 1C is a cross-sectional view of the blood collection system, illustrating an example rotary element in a second position, according to some embodiments;
Figure 1D is an upper perspective view of the rotary element, according to some embodiments;
Figure 1E is an upper perspective view of another rotary element, according to some embodiments;
Figure 1F is a cross-sectional view of the rotary element, according to some embodiments;
Figure 2A is an upper perspective view of the blood collection system, illustrating an example roller clamp, according to some embodiments;
Figure 2B is a cross-sectional view of the roller clamp, according to some embodiments;
Figure 2C is an upper perspective view of example tubing, according to some embodiments;
Figure 3A is a cross-sectional view of an example slide valve, illustrating a body of the slide valve in a first position, according to some embodiments;
Figure 3B is an upper perspective view of the slide valve, illustrating the body of the slide valve in the first position, according to some embodiments;
Figure 3C is a cross-sectional view of the slide valve, illustrating the body of the slide valve in a second position, according to some embodiments;
Figure 3D is an upper perspective view of the slide valve, illustrating the body of the slide valve in the second position, according to some embodiments;
Figure 3E is an upper perspective view of the slide valve, according to some embodiments;
Figure 4A is an upper perspective view of the blood collection system, illustrating an example slide clamp, according to some embodiments;
Figure 4B is a cross-sectional view of the slide clamp in a first position, according to some embodiments;
Figure 4C is a cross-sectional view of the slide clamp in a second position, according to some embodiments;
Figure 5A is an upper perspective view of the blood collection system, illustrating an example stopcock, according to some embodiments;
Figure 5B is a cross-sectional view of the stopcock in a first position, according to some embodiments;
Figure 5C is a cross-sectional view of the stopcock in a second position, according to some embodiments;
Figure 5D is a cross-sectional view of the stopcock, according to some embodiments;
Figure 6A is an upper perspective view of the blood collection system, illustrating an example pinch clamp, according to some embodiments;
Figure 6B is a cross-sectional view of the pinch clamp in a first position, according to some embodiments;
Figure 6C is a cross-sectional view of the pinch clamp in a second position, according to some embodiments;
Figure 6D is a cross-sectional view of the pinch clamp, according to some embodiments;
Figure 7A is an upper perspective view of another blood collection system, according to some embodiments;
Figure 7B is a cross-sectional view of the other blood collection system, according to some embodiments;
Figure 8A is an upper perspective view of another blood collection system, according to some embodiments;
Figure 8B is a lower perspective view of an example blood collection tube holder, according to some embodiments;
Figure 8C is a side view of the blood collection tube holder, according to some embodiments;
Figure 8D is another side view of the blood collection tube holder, according to some embodiments;
Figure 8E is a cross-sectional view of the blood collection tube holder along the line 8E-8E of Figure 8C, according to some embodiments;
Figure 8F is a cross-sectional view of the blood collection tube holder along the line 8F-8F of Figure 8D, illustrating an example button in a first position, according to some embodiments;
Figure 8G is a cross-sectional view of the blood collection tube holder along the line 8F-8F of Figure 8D, illustrating the button in a second position, according to some embodiments;
Figure 8H is an upper perspective view of the button, according to some embodiments;
Figure 8I is a lower perspective view of an example body of the blood collection holder, according to some embodiments; and
Figure 8J is a lower perspective view of an example tubing and an example needle coupled to the tubing, according to some embodiments.

### DESCRIPTION OF EMBODIMENTS

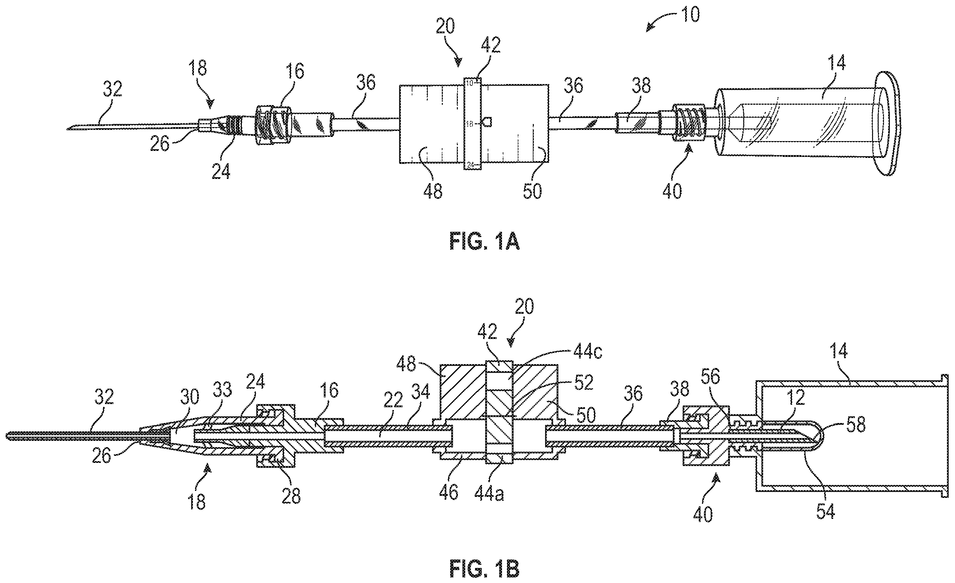

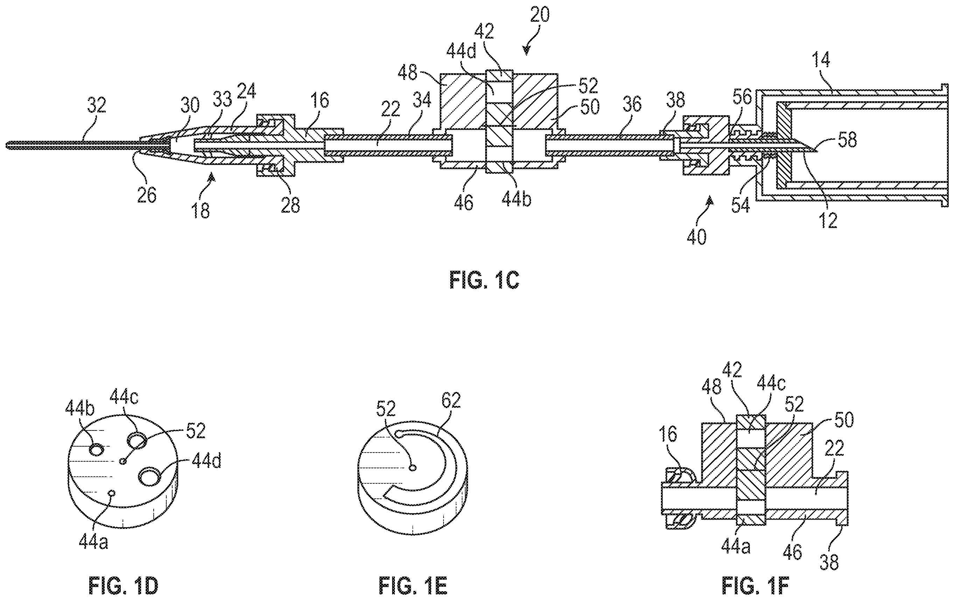

Referring now to Figures 1A-1D, in some embodiments, a blood collection system 10 may include a needle 12 configured to receive an evacuated blood collection tube. In some embodiments, the blood collection system 10 may include a blood collection tube receptacle 14, which may surround the needle 12. In some embodiments, the blood collection system 10 may include an adapter 16 configured to couple to a catheter assembly 18. In some embodiments, the blood collection system 10 may include a flow regulator 20 disposed between the needle 12 and the adapter 16. In some embodiments, the flow regulator 20 may be configured to regulate flow through a fluid pathway 22 of the blood collection system 10, which may extend between the needle 12 and the adapter 16.

In some embodiments, the adapter 16 may be coupled to the catheter assembly 18. In some embodiments, the adapter 16 may include a luer adapter, which may include a luer lock or luer slip connector. In some embodiments, the luer adapter may include a male or female luer connector. In some embodiments, the catheter assembly 18 may include a catheter adapter 24, which may include a distal end 26, a proximal end 28, and a lumen 30 extending through the distal end 26 of the catheter adapter 24 and the proximal end 28 of the catheter adapter 24. In some embodiments, the catheter assembly 18 may include a catheter 32 extending distally from the distal end 26 of the catheter adapter 24. In some embodiments, the catheter 32 may be inserted into vein of a patient. In some embodiments, in response to coupling of the adapter 16 to the catheter adapter 24, a septum 33 disposed within the lumen 30 may be penetrated.

In some embodiments, the catheter 32 may include a PIVC, such as, for example, that of the BD NEXIVA^{™} Closed IV Catheter system, the BD CATHENA^{™} Catheter system, the BD VENFLON^{™} Pro Safely Shielded IV Catheter system, the BD NEOFLON^{™} IV Cannula system, the BD INSYTE^{™} AUTOGUARD^{™} BC Shielded IV Catheter system, or another suitable peripheral intravenous catheter system. In some embodiments, the catheter 32 may include a PICC or a midline catheter. In some embodiments, the adapter 16 may be coupled to the catheter adapter 24 in any number of suitable ways. For example, the adapter 16 may be coupled to the distal end 26 of the catheter adapter 24. As another example, the adapter 16 may be coupled to an extension tube extending outwardly from the catheter adapter 24.

In some embodiments, a first tubing 34 may extend between the adapter 16 and the flow regulator 20, and a second tubing 36 may extend between the flow regulator 20 and another adapter 38, which may be configured to couple to a needle assembly 40 that includes the needle 12. In some embodiments, the first tubing 34 and/or the second tubing 36 may increase flexibility and decrease a risk of disturbing an insertion site of the catheter 32 into the patient. In some embodiments, the first tubing 34 and/or the second tubing 36 may be coupled to and/or integrated with the flow regulator 20.

In some embodiments, the flow regulator 20 may be adjusted to reduce an inner diameter of a portion of the fluid pathway 22 extending through the flow regulator 20 to be less than an inner diameter of the catheter 32. In some embodiments, after adjusting the flow regulator 20 to reduce the inner diameter of the portion of the fluid pathway 22, the needle 12 may be inserted into the evacuated blood collection tube, which may be evacuated such that a pressure within the evacuated blood collection tube is lower than ambient or atmospheric pressure.

In some embodiments, when the evacuated blood collection tube is coupled to the blood collection system 10 via insertion of the needle 12, blood may flow more slowly than it otherwise would due to the reduced inner diameter and restriction of the fluid pathway. Generally, when the evacuated blood collection tube is coupled to a blood collection system, a high pressure differential between the evacuated blood collection tube and the vein creates a risk of hemolysis. However, in some embodiments, the decrease of blood flow due to the reduced inner diameter and restriction of the fluid pathway 22 may decrease the risk of hemolysis. In some embodiments, the decrease of blood flow may also reduce a risk of collapse of the vein and/or the catheter 32.

In some embodiments, as the evacuated blood collection tube fills with blood, a vacuum within the evacuated blood collection tube decreases and the pressure differential between the evacuated blood collection tube and the vein decreases. In some embodiments, the decreased pressure differential may result in the evacuated blood collection tube filling more slowly over time. In some embodiments, in response to the evacuated blood collection tube partially filling with blood, the flow regulator 20 may be adjusted to increase the inner diameter of the fluid pathway 22 extending through the flow regulator 20, which may increase blood flow and speed blood collection. In some embodiments, the inner diameter may be increased to be larger than the inner diameter when the needle 12 is inserted into the evacuated blood collection tube. In some embodiments, the inner diameter may be increased to be equal to (or greater) than the inner diameter of the catheter 32.

In some embodiments, the flow regulator 20 may include a rotary element 42. In some embodiments, in response to the rotary element 42 being in a first position, a portion of the fluid pathway 22 of the blood collection system 10 may extend through the rotary element 42. An example of the first position is illustrated in Figure 1B. In some embodiments, in response to rotation of the rotary element 42 from the first position to a second position, a diameter of the portion of the fluid pathway 22 extending through the rotary element 42 may change. An example of the second position is illustrated in Figure 1C. In some embodiments, the rotary element 42 may include one or more holes 44 configured to align with the fluid pathway 22 of the blood collection system 10. In some embodiments, each of the holes 44 may have a different diameter and/or size.

In some embodiments, when the rotary element 42 is in the first position, a first hole 44a may be aligned with the fluid pathway 22, and when the rotary element 42 is in the second position, a second hole 44b may be aligned with the fluid pathway 22. In some embodiments, the first hole 44a may be smaller than the second hole 44b and smaller than the inner diameter of the catheter 32. In some embodiments, a size or inner diameter of the second hole 44b may be between the first hole 44a and the catheter 32. In some embodiments, the size or inner diameter of the second hole 44b may equal to the catheter 32.

In some embodiments, the rotary element 42 may be in the first position prior to coupling the evacuated blood collection tube to the blood collection system 10 or immediately after coupling the evacuated blood collection tube to the blood collection system. In some embodiments, in response to the evacuated blood collection tube partially filling with blood, the user may rotate the rotary element 42 to the second position. In some embodiments, the first position may correspond to a closed or partially restricted state. In some embodiments, the second position may facilitate a higher flow rate that the first position.

In some embodiments, the rotary element 42 may be rotated with respect to a body 46 of the flow regulator 20, which may include a distal piece 48 and/or a proximal piece 50. In some embodiments, the rotary element 42 may be coupled to the distal piece 48 and/or the proximal piece 50 via a pin 52. In some embodiments, the pin 52 may extend through the distal piece 48, the rotary element 42, and/or the proximal piece 50. In some embodiments, the rotary element 42 may rotate about the pin 52. In some embodiments, the rotary element 42 may be coupled to the distal piece 48 and/or the proximal piece 50 via any number of suitable ways.

In some embodiments, the needle assembly 40 may include a luer adapter, which may include a luer lock or luer slip connector. In some embodiments, the luer adapter may include a male or female luer connector. In some embodiments, the needle 12 may extend proximally from the luer adapter. In some embodiments, the needle assembly 40 may include one or more threads, which may be configured to couple to the blood collection tube receptacle 14, which may be generally cylindrical.

In some embodiments, an elastomeric sheath 54 may be coupled to the needle assembly 40. In some embodiments, and a proximal end 56 of the needle 12 may be enveloped within the elastomeric sheath 54. In some embodiments, the elastomeric sheath 54 may include an open distal end 58 and a closed proximal end 60. In some embodiments, in response to the evacuated blood collection tube pushing the elastomeric sheath 54 distally, the needle 12 may pierce the elastomeric sheath 54, and the needle 12 may insert into a cavity of the evacuated blood collection tube.

In some embodiments, the rotary element 42 may include one or more markings. In some embodiments, the markings may each indicate a catheter gauge size, such as, for example, 18g, 20g, 22g, or 24g. In some embodiments, the distal piece 48 or the proximal piece 50 may include another marking configured to align with the markings of the rotary element 42. In some embodiments, the user may align a particular marking of the rotary element 42 that indicates a particular gauge size with the other marking when the catheter 32 being used is the particular gauge size. In some embodiments, in response to alignment of the particular marking of the rotary element 42 with the other marking, a particular hole 44 may be aligned with the fluid pathway 22. In some embodiments, the particular hole 44 may include a smaller inner diameter than the particular gauge size.

Referring now to Figure 1E, in some embodiments, a rotary element 61 may include a curved slot 62 extending through the rotary element 61. In some embodiments, the rotary element 61 may be similar or identical to the rotary element 42 discussed with respect to Figures 1A-1D in terms of one or more included features and/or operation. In some embodiments, an entirety of the curved slot 62 may be configured to align with the fluid pathway 22 of the blood collection system 10 through rotation of the rotary element 61. In some embodiments, an inner diameter or width of the curved slot 62 may continuously increase such that fluid flow through the rotary element 61 continuously changes as the rotary element 61 is rotated.

In some embodiments, the rotary element 61 may be in a first position prior to coupling the evacuated blood collection tube to the blood collection system 10 or immediately after coupling the evacuated blood collection tube to the blood collection system. In some embodiments, in response to the evacuated blood collection tube partially filling with blood, the user may rotate the rotary element 61 to the second position. In some embodiments, when the rotary element 61 is in the first position, a portion of the curved slot 62 with a first inner diameter may be aligned with the fluid pathway 22. In some embodiments, when the rotary element 61 is in the second position, another portion of the curved slot 62 with a second inner diameter may be aligned with the fluid pathway 22. In some embodiments, the second inner diameter may be greater than the first inner diameter. In some embodiments, the first position may correspond to a closed or partially restricted state. In some embodiments, the second position may facilitate a higher flow rate that the first position.

Referring now to Figure 1F, in some embodiments, the blood collection system 10 may not include the first tubing 34 and/or the second tubing 36. In some embodiments, the adapter 16 and/or the other adapter 38 may be integrally formed with the flow regulator 20.

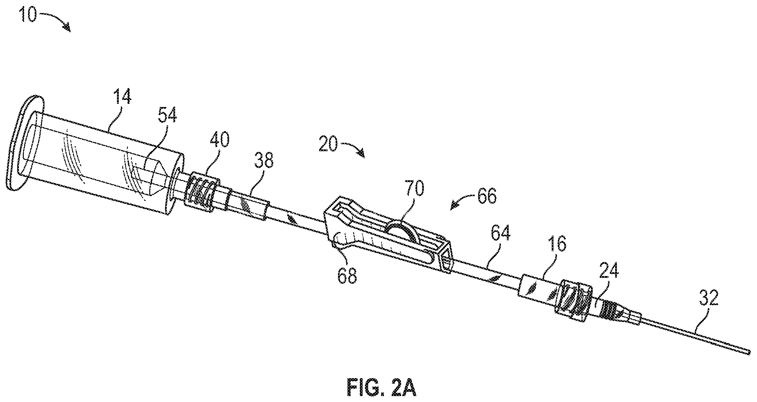

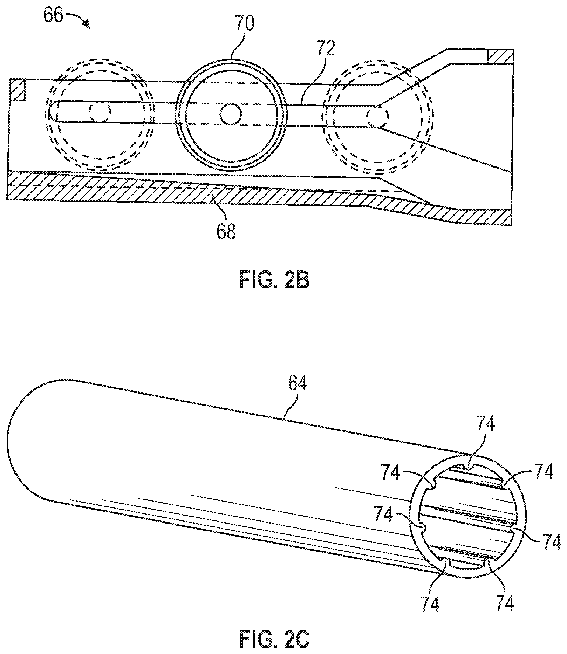

Referring now to Figures 2A-2B, in some embodiments, the blood collection system 10 may include a tubing 64, which may extend between the adapter 16 and the needle 12. In some embodiments, the tubing 64 may be elastically deformable. In some embodiments, the flow regulator 20 may include a clamp disposed on the tubing 64. In some embodiments, the clamp on the tubing 64 may include a roller clamp 66. In some embodiments, the roller clamp 66 may include any suitable roller clamp known in the art.

In some embodiments, the roller clamp 66 may include a substantially rigid elongated frame 68, a generally cylindrical roller 70, and a length of the tubing 64 disposed within the frame. In some embodiments, a roller track 72 or the frame 68 may be inclined. In some embodiments, a flow rate through the tubing 64 may be controlled by moving the generally cylindrical roller 70 along the roller track 72 and over the tubing 64, thereby selectively compressing tubing 64 to attain a desired flow rate.

In some embodiments, the generally cylindrical roller 70 may be in a closed or partially restricted state prior to coupling the evacuated blood collection tube to the blood collection system 10 or immediately after coupling the evacuated blood collection tube to the blood collection system 10. In some embodiments, in response to the evacuated blood collection tube partially filling with blood, the generally cylindrical roller 70 may be moved by the user from the closed or partially restricted state to a high flow state.

Referring now to Figure 2C, in some embodiments, an inner surface of the tubing 64 may include one or more ribs 74 or protrusions, which may extend generally parallel to a longitudinal axis of the tubing 64. In some embodiments, the ribs 74 may prevent complete occlusion of the tubing 64. In some embodiments, the ribs 74 may maintain a minimum flow rate through the tubing 64 even when the tubing 64 is clamped or pinched. In some embodiments, the ribs 74 may extend through a portion of the tubing 64 disposed within the flow regulator 20. In some embodiments, the ribs 74 may extend along all or a portion of the tubing 64.

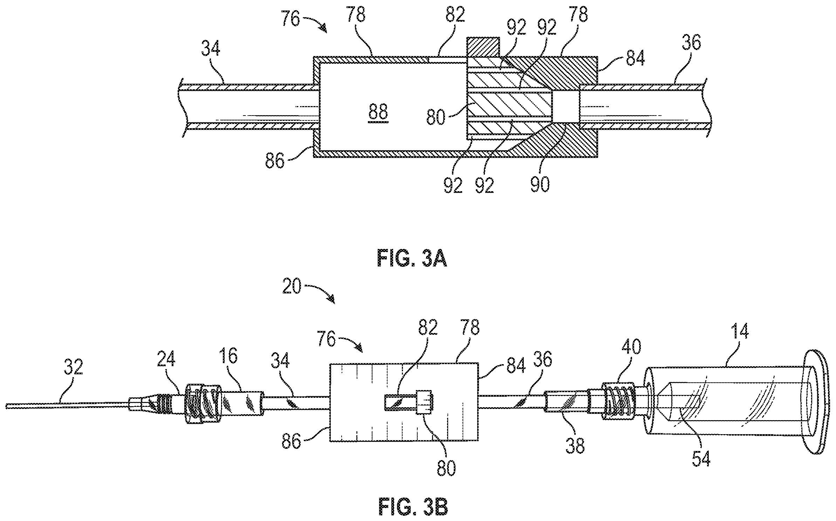

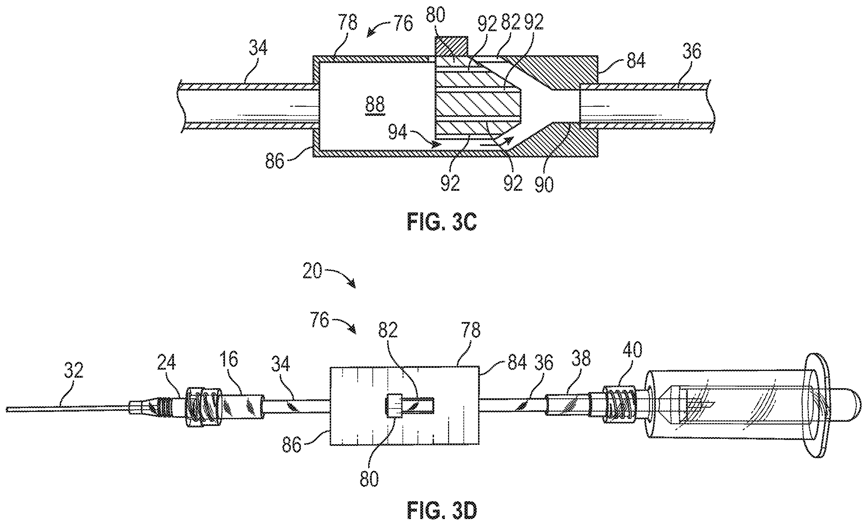

Referring now to Figures 3A-3E, in some embodiments, the flow regulator 20 may include a slide valve 76. In some embodiments, the slide valve 76 may include a housing 78 and a body 80 slidable with respect to the housing 78 between a first position and a second position. In some embodiments, the housing 78 may include a slot 82, and the body 80 may extend through the slot 82 for gripping by the user.

In some embodiments, the housing 78 may include a first end 84, a second end 86, and a lumen 88 extending through the first end 84 and the second end 86. In some embodiments, the first end 84 of the housing 78 may include an opening 90. In some embodiments, as illustrated, the first end 84 may be proximal to the second end 86 and the body 80 may move proximally to close the opening 90. In some embodiments, the first end 84 may be distal to the second end 86, and the body 80 may move distally to close the opening 90.

In some embodiments, an outer surface of the body 80 may include one or more channels 92. In some embodiments, in response to the body 80 being in the first position, illustrated, for example, in Figure 3A, blood may be configured to flow through a gap 94 between an outer surface of the body 80 and the housing 78 and through the opening 90. In some embodiments, in response to the housing 78 being in the second position, illustrated, for example, in Figure 3C, blood may be configured to flow through at least one of the channels 92 and through the opening 90 but may not flow through the gap 94.

In some embodiments, the body 80 may be in the first position prior to coupling the evacuated blood collection tube to the blood collection system 10 or immediately after coupling the evacuated blood collection tube to the blood collection system 10. In some embodiments, in response to the evacuated blood collection tube partially filling with blood, the body 80 may be moved by the user from first position to the second position. In some embodiments, the first position may correspond to a closed or partially restricted state. In some embodiments, the second position may facilitate a higher flow rate that the first position.

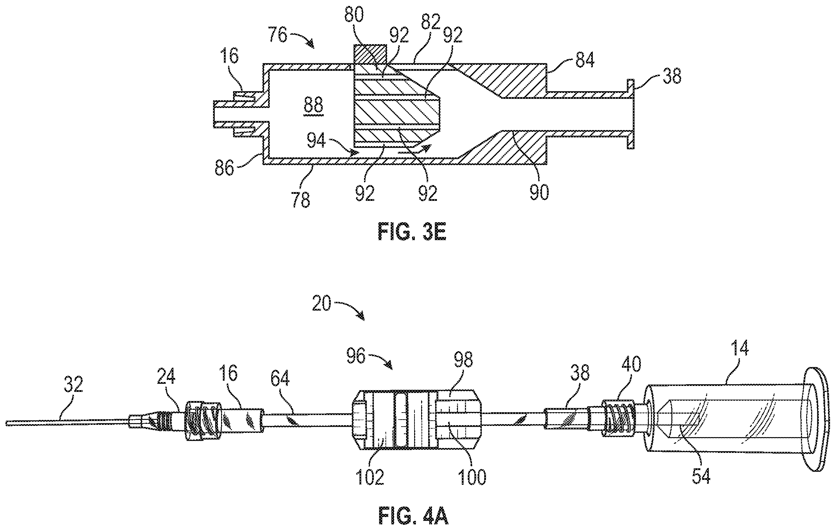

Referring now to Figure 3E, in some embodiments, the blood collection system 10 may not include the first tubing 34 and/or the second tubing 36. In some embodiments, the adapter 16 and/or the other adapter 38 may be integrally formed with the flow regulator 20.

Referring now to Figures 4A-4C, in some embodiments, the flow regulator 20 may include a slide valve 76. In some embodiments, the blood collection system 10 may include the tubing 64, which may extend between the adapter 16 and the needle 12. In some embodiments, the flow regulator 20 may include a clamp disposed on the tubing 64. In some embodiments, the clamp on the tubing 64 may include a slide clamp 96.

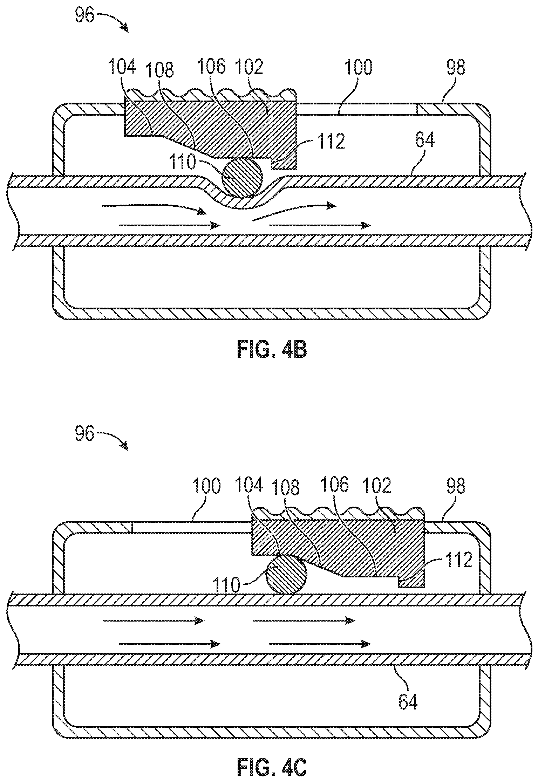

In some embodiments, the slide clamp 96 may include a housing 98, which may include a slot 100. In some embodiments, the slide clamp 96 may include a body 102, which may extend through the slot 100. In some embodiments, the body 80 may be slidable with respect to the housing 98 between a first position, illustrated, for example, in Figure 4B, and a second position, illustrated, for example, in Figure 4C. In some embodiments, a bottom of the body 80 may include a first surface 104, a second surface 106, and a third surface 108 disposed between the first surface 104 and the second surface 106. In some embodiments, the first surface 104 and the second surface 106 may be planar and/or generally parallel to the longitudinal axis of the tubing 64. In some embodiments, the third surface 108 may be angled with respect to the longitudinal axis of the tubing 64. In some embodiments, the second surface 106 may be closer to the tubing 64 than the first surface 104.

In some embodiments, the slide clamp 96 may include a roller pin 110 disposed between the body 102 and the tubing 64. In some embodiments, in response to the body 102 being in the first position, the roller pin 110 may contact the first surface 104 and the tubing 64. In some embodiments, in response to the body 102 sliding from the first position to the second position, the roller pin 110 moves or rolls from the first surface 104 along the third surface 108 to the second surface 106 and decreases fluid flow through the tubing 64. In some embodiments, a fourth surface 112 or stop surface may be disposed at an end of the body 102 and may prevent the roller pin 110 from rolling beyond the end of the body 102.

In some embodiments, the body 102 may be in the first position prior to coupling the evacuated blood collection tube to the blood collection system 10 or immediately after coupling the evacuated blood collection tube to the blood collection system 10. In some embodiments, in response to the evacuated blood collection tube partially filling with blood, the body 102 may be moved by the user from first position to the second position. In some embodiments, the first position may correspond to a closed or partially restricted state. In some embodiments, the second position may facilitate a higher flow rate that the first position.

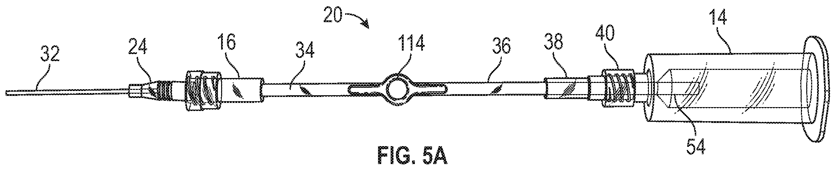

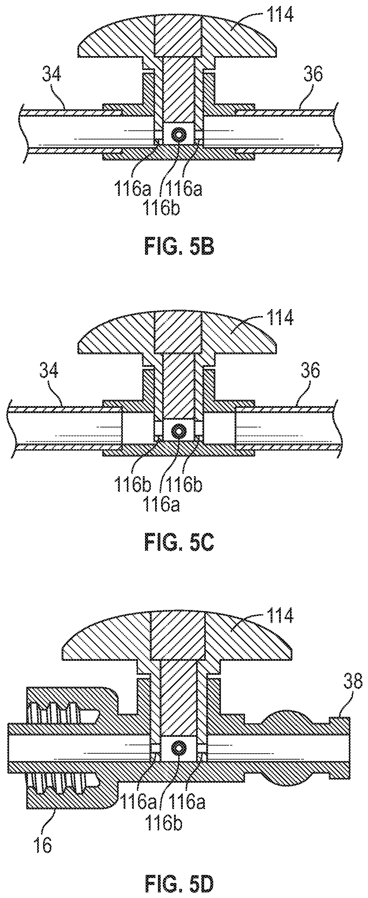

Referring now to Figures 5A-5C, in some embodiments, the flow regulator 20 may include a stopcock 114. In some embodiments, the stopcock 114 may be in the first position prior to coupling the evacuated blood collection tube to the blood collection system 10 or immediately after coupling the evacuated blood collection tube to the blood collection system 10. In some embodiments, in response to the evacuated blood collection tube partially filling with blood, the stopcock 114 may be moved by the user from first position, illustrated, for example, in Figure 5B, to the second position, illustrated, for example, in Figure 5C. In some embodiments, the first position may correspond to a closed or partially restricted state. In some embodiments, the second position may facilitate a higher flow rate that the first position.

In some embodiments, in response to rotation of the stopcock 114 from the first position to the second position, a diameter of the portion of the fluid pathway 22 extending through the stopcock 114 may change. In some embodiments, the stopcock 114 may include one or more pairs of holes 116 configured to align with the fluid pathway 22 of the blood collection system 10. In some embodiments, a first hole and a second hole of the pair of holes 116 may be disposed directly across from each other. In some embodiments, each of the pairs of holes 44 may have a different diameter and/or size.

In some embodiments, when the stopcock 114 is in the first position, a first pair of holes 116a may be aligned with the fluid pathway 22, and when the stopcock 114 is in the second position, a second pair of holes 116b may be aligned with the fluid pathway 22. In some embodiments, the first pair of holes 116a may be smaller than the second pair of holes 116b and smaller than the inner diameter of the catheter 32.

Referring now to Figure 5D, in some embodiments, the blood collection system 10 may not include the first tubing 34 and/or the second tubing 36. In some embodiments, the adapter 16 and/or the other adapter 38 may be integrally formed with the flow regulator 20.

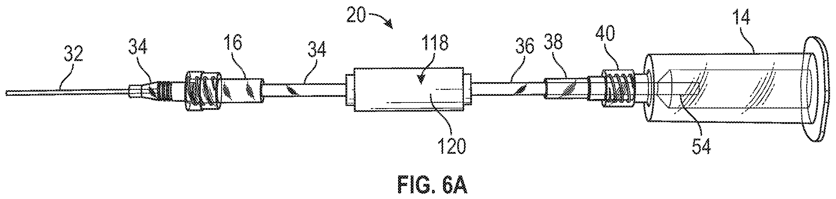

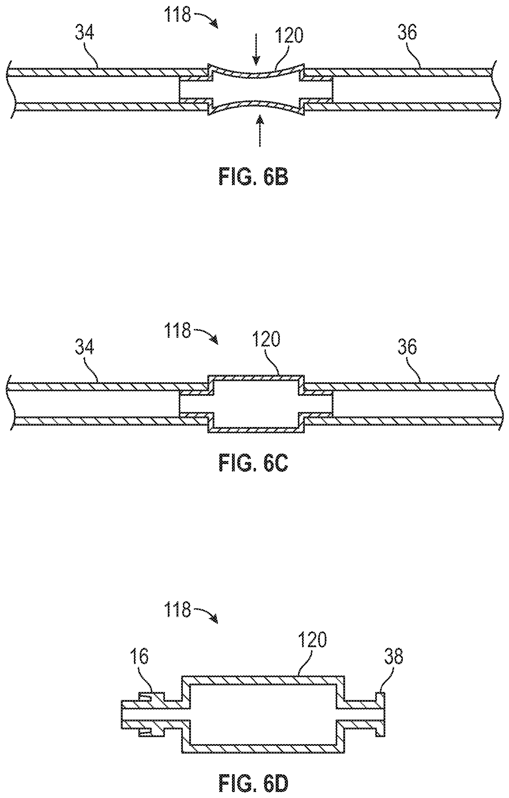

Referring now to Figures 6A-6C, in some embodiments, the flow regulator 20 may include a pinch valve 118. In some embodiments, the pinch valve 118 may include a body 120, through which the fluid pathway 22 may extend. In some embodiments, a first end of the pinch valve 118 may be coupled to the first tubing 34 and/or a second end of the pinch valve 118 may be coupled to the second tubing 36. In some embodiments, the pinch valve 118 may be elastically deformable.

In some embodiments, in response to the user pinching the body 120, the fluid pathway 22 may be closed or partially restricted. In some embodiments, the user may pinch the body 120 prior to coupling the evacuated blood collection tube to the blood collection system 10 or immediately after coupling the evacuated blood collection tube to the blood collection system 10. In some embodiments, in response to the evacuated blood collection tube partially filling with blood, the user pinch the body 120 less or cease pinching the body 120, and flow through the fluid pathway 22 may increase.

Referring now to Figure 6D, in some embodiments, the blood collection system 10 may not include the first tubing 34 and/or the second tubing 36. In some embodiments, the adapter 16 and/or the other adapter 38 may be integrally formed with the flow regulator 20.

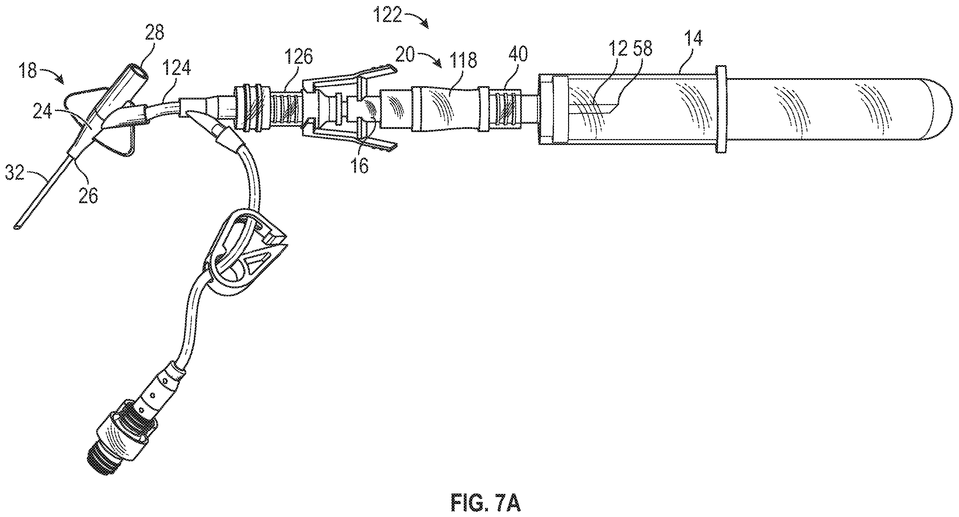

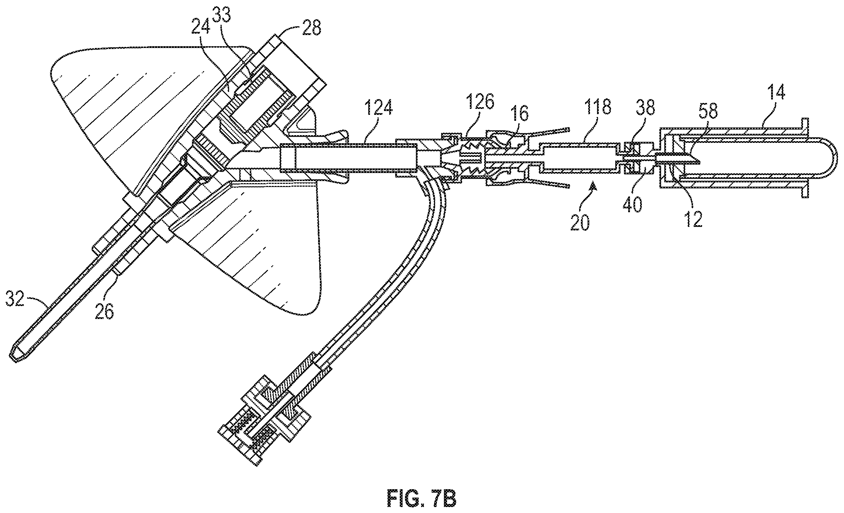

Referring to Figures 7A-7B, a blood collection system 122 is illustrated, according to some embodiments. In some embodiments, blood collection system 122 may be similar or identical to the blood collection system 10 discussed with respect to Figures 1A-6D in terms of one or more included features and/or operation. As mentioned, in some embodiments, the adapter 16 may be coupled to the catheter adapter 24 in any number of suitable ways. Figures 7A-7B illustrate the adapter 16 coupled to an extension tube 124 extending outwardly from the catheter adapter 24. In some embodiments, the adapter 16 may include a blunt cannula, which may be inserted into a needleless connector 126. Although the flow regulator 20 includes the pinch valve 118 in Figures 7A-7B, it is understood that in some embodiments, any of the flow regulators 20 of the present disclosure may be used in the blood collection system 122. In some embodiments, the first tubing 34 or the tubing 64 may extend proximally from the adapter 16 to provide increased flexibility.

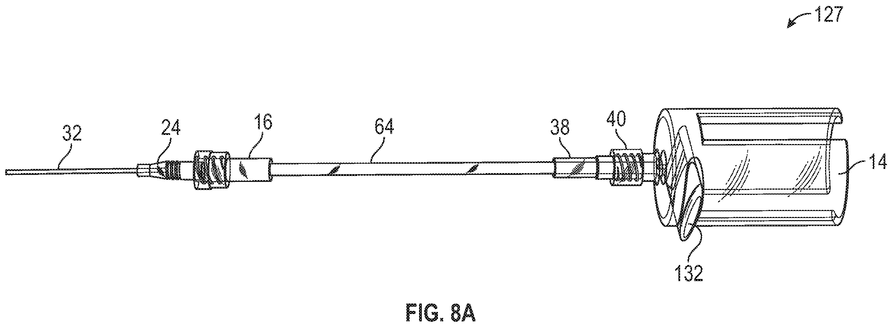

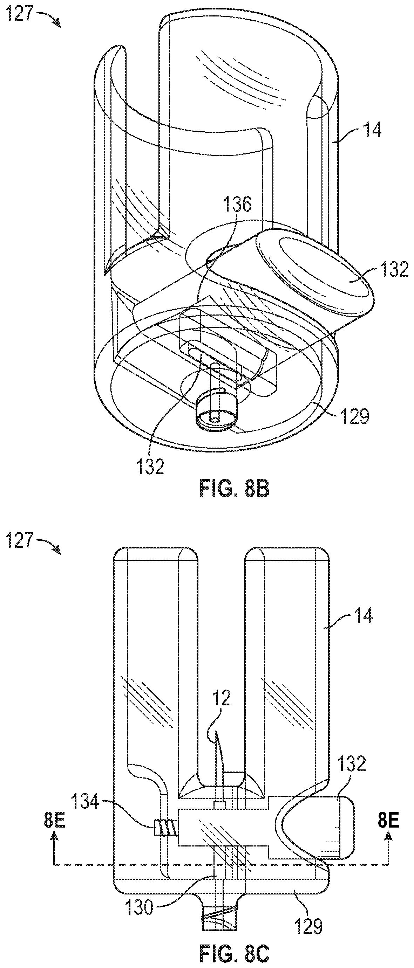

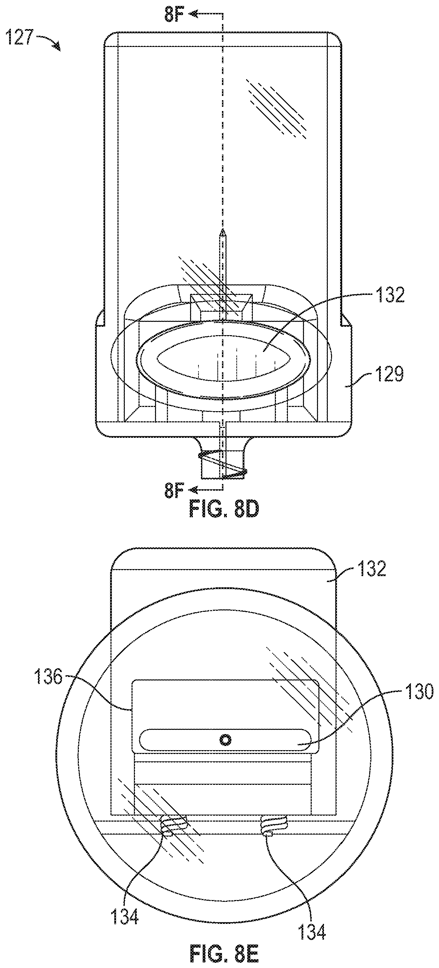

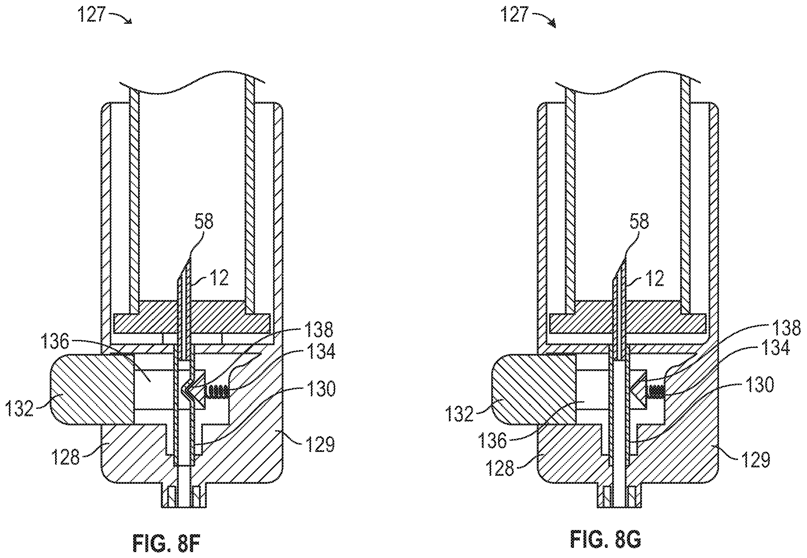

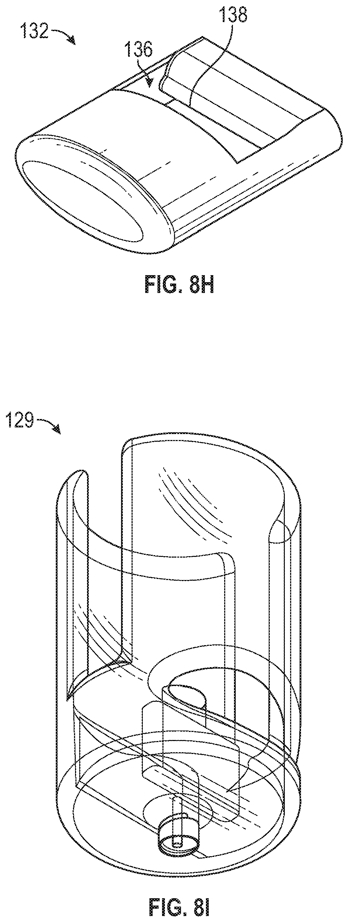

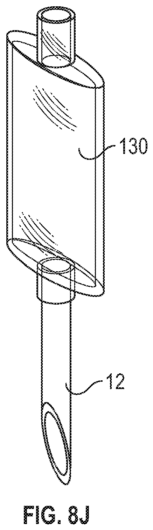

Referring now to Figures 8A-8J, in some embodiments, a blood collection tube holder 127 may include one or more of the following: the needle 12 configured to receive the evacuated collection tube; a cavity 128 of a body 129; a tubing 130 disposed within the cavity 128 and in fluid communication with the needle 12; a button 132 disposed within the cavity 128; and one or more springs 134. In some embodiments, the button 132 may include an opening 136 extending through the button 132. In some embodiments, the button 132 may include a rib 138 proximate the opening 136. In some embodiments, the tubing 130 may extend through the opening 136. In some embodiments, the body 129 may include a blood collection tube receptacle 14.

In some embodiments, the springs 134 may be disposed between the button 132 and a wall 140 of the cavity 128. In some embodiments, the springs 134 may bias the rib 138 against the tubing 130 to compress the tubing 130. In some embodiments, in response to the button 132 being depressed, the springs 134 and the tubing 130 may be compressed. In some embodiments, when the tubing 130 is compressed, the fluid pathway 22 may be closed or partially restricted. In some embodiments, in response to the evacuated blood collection tube partially filling with blood, the user may depress the button 132. In some embodiments, a distal end of the body 129 may include a luer adapter.

All examples and conditional language recited herein are intended for pedagogical objects to aid the reader in understanding the invention and the concepts contributed by the inventor to furthering the art, and are to be construed as being without limitation to such specifically recited examples and conditions. Although embodiments of the present inventions have been described in detail, it should be understood that the various changes, substitutions, and alterations could be made hereto without departing from the scope of the invention.

## Claims

1. A blood collection system (10, 122), comprising:
a needle (12) configured to receive an evacuated blood collection tube;
an adapter (16) configured to couple to a catheter assembly (18); and
a flow regulator (20) disposed between the needle (12) and the adapter (16) and configured to regulate flow through a fluid pathway (22) extending between the needle (12) and the adapter (16),
**characterized in that**:
the flow regulator (20) comprises a rotary element (42, 61), wherein in response to the rotary element (42, 61) being in a first position, a portion of the fluid pathway (22) of the blood collection system extends through the rotary element (42, 61), wherein in response to rotation of the rotary element (42, 61) from the first position to a second position, a diameter of the portion of the fluid pathway (22) extending through the rotary element (42, 61) changes.

2. The blood collection system (10, 122) of claim 1, wherein the rotary element (42) comprises a plurality of holes (44, 44a, 44b, 44c, 44d) configured to align with the fluid pathway (22) of the blood collection system, wherein each of the plurality of holes (44, 44a, 44b, 44c, 44d) has a different diameter.

3. The blood collection system (10, 122) of claim 1, wherein the rotary element (61) comprises a curved slot (62) extending through the rotary element (61), wherein an entirety of the curved slot (62) is configured to align with the fluid pathway (22) of the blood collection system, wherein a width of the curved slot (62) continuously increases such that fluid flow through the rotary element (61) continuously changes as the rotary element (61) is rotated.

4. The blood collection system (10, 122) of any one of the preceding claims, further comprising a blood collection tube receptacle (14), wherein the blood collection tube receptacle (14) surrounds the needle (12).

5. The blood collection system (10, 122) of any one of the preceding claims, further comprising the catheter assembly (18), wherein the catheter assembly (18) comprises:
a catheter adapter (24), comprising a distal end (26), a proximal end (28), and a lumen (30) extending through the distal end (26) of the catheter adapter (24) and the proximal end (28) of the catheter adapter (24); and
a catheter (32) extending distally from the distal end (26) of the catheter adapter (24).

## Patentansprüche

1. Blutentnahmesystem (10, 122), das aufweist:
eine Nadel (12), die zur Aufnahme eines evakuierten Blutentnahmeröhrchens ausgebildet ist;
einen Adapter (16), der so ausgebildet ist, dass er mit einer Katheterbaugruppe (18) gekoppelt wird; und
einen Durchflussregler (20), der zwischen der Nadel (12) und dem Adapter (16) angeordnet und so ausgebildet ist, dass er den Durchfluss durch einen Fluidweg (22), der sich zwischen der Nadel (12) und dem Adapter (16) erstreckt, reguliert, **dadurch gekennzeichnet, dass**
der Durchflussregler (20) ein Drehelement (42, 61) aufweist, wobei sich als Reaktion darauf, dass sich das Drehelement (42, 61) in einer ersten Position befindet, ein Teil des Fluidwegs (22) des Blutentnahmesystems durch das Drehelement (42, 61) erstreckt, wobei sich als Reaktion auf eine Drehung des Drehelements (42, 61) von der ersten Position zu einer zweiten Position ein Durchmesser des Teils des Fluidwegs (22), der sich durch das Drehelement (42, 61) erstreckt, ändert.

2. Blutentnahmesystem (10, 122) nach Anspruch 1, wobei das Drehelement (42) eine Vielzahl von Löchern (44, 44a, 44b, 44c, 44d) aufweist, die so ausgebildet sind, dass sie mit dem Fluidweg (22) des Blutentnahmesystems ausgerichtet sind, wobei jedes der Vielzahl von Löchern (44, 44a, 44b, 44c, 44d) einen anderen Durchmesser aufweist.

3. Blutentnahmesystem (10, 122) nach Anspruch 1, wobei das Drehelement (61) einen gekrümmten Spalt (62) aufweist, der sich durch das Drehelement (61) erstreckt, wobei eine Gesamtheit des gekrümmten Spalts (62) so ausgebildet ist, dass er mit dem Fluidweg (22) des Blutentnahmesystems ausgerichtet ist, wobei eine Breite des gekrümmten Spalts (62) kontinuierlich zunimmt, so dass sich der Fluidstrom durch das Drehelement (61) kontinuierlich ändert, wenn das Drehelement (61) gedreht wird.

4. Blutentnahmesystem (10, 122) nach einem der vorstehenden Ansprüche, das ferner eine Blutentnahmeröhrchenaufnahme (14) aufweist, wobei die Blutentnahmeröhrchenaufnahme (14) die Nadel (12) umgibt.

5. Blutentnahmesystem (10, 122) nach einem der vorhergehenden Ansprüche, das ferner die Katheterbaugruppe (18) aufweist, wobei die Katheterbaugruppe (18) aufweist:
einen Katheteradapter (24), der ein distales Ende (26), ein proximales Ende (28) und ein Lumen (30) aufweist, das sich durch das distale Ende (26) des Katheteradapters (24) und das proximale Ende (28) des Katheteradapters (24) erstreckt; und
einen Katheter (32), der sich distal von dem distalen Ende (26) des Katheteradapters (24) erstreckt.

## Revendications

1. Système de prélèvement de sang (10, 122), comprenant :
une aiguille (12) configurée pour recevoir un tube de prélèvement de sang sous vide ;
un adaptateur (16) configuré pour se coupler à un ensemble cathéter (18); et
un régulateur de flux (20) disposé entre l'aiguille (12) et l'adaptateur (16) et configuré pour réguler le flux à travers un trajet de fluide (22) s'étendant entre l'aiguille (12) et l'adaptateur (16),
**caractérisé en ce que** :
le régulateur de flux (20) comprend un élément rotatif (42, 61), où, en réponse à l'élément rotatif (42, 61) se trouvant dans une première position, une partie du trajet de fluide (22) du système de prélèvement de sang s'étend à travers l'élément rotatif (42, 61), où, en réponse à la rotation de l'élément rotatif (42, 61) de la première position à une seconde position, un diamètre de la partie du trajet de fluide (22) s'étendant à travers l'élément rotatif (42, 61) change.

2. Système de prélèvement de sang (10, 122) selon la revendication 1, dans lequel l'élément rotatif (42) comprend une pluralité de trous (44, 44a, 44b, 44c, 44d) configurés pour s'aligner avec le trajet de fluide (22) du système de prélèvement de sang, où chacun de la pluralité de trous (44, 44a, 44b, 44c, 44d) a un diamètre différent.

3. Système de prélèvement de sang (10, 122) selon la revendication 1, dans lequel l'élément rotatif (61) comprend une fente incurvée (62) s'étendant à travers l'élément rotatif (61), où l'ensemble de la fente incurvée (62) est configuré pour s'aligner avec le trajet de fluide (22) du système de prélèvement de sang, où la largeur de la fente incurvée (62) augmente de manière continue de sorte que le flux de fluide à travers l'élément rotatif (61) change de manière continue à mesure que l'élément rotatif (61) est mis en rotation.

4. Système de prélèvement de sang (10, 122) selon l'une quelconque des revendications précédentes, comprenant en outre un réceptacle de tube de prélèvement de sang (14), où le réceptacle de tube de prélèvement de sang (14) entoure l'aiguille (12).

5. Système de prélèvement de sang (10, 122) selon l'une quelconque des revendications précédentes, comprenant en outre l'ensemble cathéter (18), où l'ensemble cathéter (18) comprend :
un adaptateur de cathéter (24), comprenant une extrémité distale (26), une extrémité proximale (28) et une lumière (30) s'étendant à travers l'extrémité distale (26) de l'adaptateur de cathéter (24) et l'extrémité proximale (28) de l'adaptateur de cathéter (24); et
un cathéter (32) s'étendant de manière distale depuis l'extrémité distale (26) de l'adaptateur de cathéter (24).
